(19) Europäisches Patentamt
European Patent Office
Office européen des brevets

(11) EP 4 159 215 A1

(12) EUROPEAN PATENT APPLICATION
published in accordance with Art. 153(4) EPC

(43) Date of publication:
**05.04.2023 Bulletin 2023/14**

(21) Application number: **21814378.2**

(22) Date of filing: **26.05.2021**

(51) International Patent Classification (IPC):
***A61K 31/4965*** *(2006.01)* ***A61K 31/7076*** *(2006.01)*
***A61P 31/14*** *(2006.01)* ***A61P 43/00*** *(2006.01)*
***C07D 241/24*** *(2006.01)*

(52) Cooperative Patent Classification (CPC):
**A61K 31/4965; A61K 31/7076; A61P 31/14; A61P 43/00; C07D 241/24;** Y02P 20/55

(86) International application number:
**PCT/JP2021/019918**

(87) International publication number:
**WO 2021/241613 (02.12.2021 Gazette 2021/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.05.2020 JP 2020091891**

(71) Applicant: **FUJIFILM Toyama Chemical Co., Ltd.**
**Chuo-ku**
**Tokyo 104-0031 (JP)**

(72) Inventors:
- **KOMENO Takashi**
  **Toyama-shi, Toyama 930-8508 (JP)**
- **YAMASHITA Nozomi**
  **Toyama-shi, Toyama 930-8508 (JP)**
- **KUROSAKI Chie**
  **Toyama-shi, Toyama 930-8508 (JP)**

(74) Representative: **Hoffmann Eitle**
**Patent- und Rechtsanwälte PartmbB**
**Arabellastraße 30**
**81925 München (DE)**

# (54) THERAPEUTIC AGENT FOR RNA VIRAL INFECTION OBTAINED BY COMBINING PYRAZINE DERIVATIVE AND THIOPURINE DERIVATIVE

(57) The present invention addresses the problem of providing a therapeutic agent for RNA viral infection that includes a novel combination of a pyrazine derivative and a specific compound, the novel combination exhibiting an effect against the RNA virus. The present invention also addresses the problem of providing a therapeutic agent for RNA viral infection that includes a combination of a pyrazine derivative and a specific compound, the combination being capable of simultaneously enhancing anti-virus activities against multiple RNA viruses. The present invention provides a therapeutic agent for RNA viral infection obtained by combining a pyrazine derivative or a salt thereof and a thiopurine derivative.

EP 4 159 215 A1

## Description

### TECHNICAL FIELD

The present invention relates to a therapeutic agent for an RNA virus infection comprising a combination of a pyrazine derivative or a salt thereof and a thiopurine derivative.

### BACKGROUND TECHNOLOGY

RNA viruses such as influenza virus and Ebola virus cause various infectious diseases, and their corresponding measures are required. On the other hand, a pyrazine derivative is known as a compound having a wide antiviral activity against many RNA viruses (hereinafter also referred to as T -705) (Non-Patent Document 1).

If the activity of the pyrazine derivative can be enhanced, it is more useful as a medicine. Thus, it has been reported that a synergistic effect on influenza viruses is exhibited by combining the Favipiravir and the neuraminidase inhibitor (Patent Document 1). Also, it has been reported that a synergistic effect on the Ebola virus is exhibited by combining the Favipiravir and Gemcitabine or Obatoclax (Patent Document 2).

However, the RNA virus acquires drug resistance by evolution. For example, a neuraminidase inhibitor-resistant influenza virus is known (Non-Patent Document 2). In order to cope with such resistance acquisition, a new combination of compounds exhibiting an effect on the RNA virus is always required.

As described above, it is a characteristic of Favipiravir that it has a wide range of effects on many RNA viruses. However, a combination of a pyrazine derivative and a specific compound capable of simultaneously enhancing antiviral activity against a plurality of RNA viruses is not known.

It has been known that 6-methylmercaptopurine riboside, which is a thiopurine derivative, has a certain antiviral activity (Non-Patent Document 3). However, it has not been known that what kind of action is shown in the cells when used in combination with the Favipiravir.

### PRIOR ART DOCUMENT

### PATENT DOCUMENT

Patent Document 1: International Publication No. 08/099874
Patent Document 2: International Publication No. 2007/202789

### NON-PATENT DOCUMENT

Non-Patent Document 1: Proc Jpn Acad Ser B Phys Biol Sci 93, 449-463
Non-Patent Document 2: CDC Health Advisory : CDC issues interim recommendations for the use of influenza antivirals in the setting of oseltamivir Resistance among circulating influenza A (H1N1) viruses, 2008-09 Season.
Non-Patent Document 3: PLoS One. October 2011, Volume 6, Issue 10, e26697

### SUMMARY OF INVENTIONPROBLEM TO BE SOLVED BY THE INVENTION

An object of the present invention is to provide a therapeutic agent for an RNA virus infection comprising a new combination of a pyrazine derivative and a specific compound showing an effect on an RNA virus. Another object of the present invention is to provide a therapeutic agent for an RNA virus infection comprising a combination of a pyrazine derivative and a specific compound capable of simultaneously enhancing antiviral activity against a plurality of RNA viruses.

### MEANS FOR SOLVING THE PROBLEM

As a result of intensive studies by the present inventors under such a situation, the present inventors found that when the pyrazine derivative or a salt thereof represented by a formula [1]

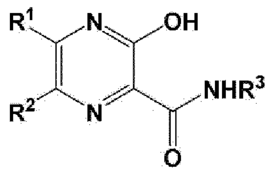

[1]

(In the formula, $R^1$ and $R^2$ are the same or different and represent a hydrogen atom or a halogen atom; and $R^3$ represents a hydrogen atom or an amino protecting group.) and a thiopurine derivative such as 6-methylmercaptopurine riboside are used in combination, the antiviral activity against the RNA virus is enhanced, and the present inventors found that at least a part of the enhancing action is caused by an increase in the amount of the pyrazine derivative ribose triphosphate in the cell. The present inventors completed the present invention.

That is, the present invention provides the following.

[1] A therapeutic agent for an RNA virus infection comprising a combination of a pyrazine derivative or a salt thereof represented by a formula [1]

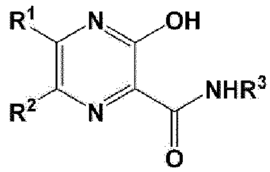

[1]

(In the formula, $R^1$ and $R^2$ are the same or different and represent a hydrogen atom or a halogen atom; and $R^3$ represents a hydrogen atom or an amino protecting group.) and a thiopurine derivative represented by a formula [2]

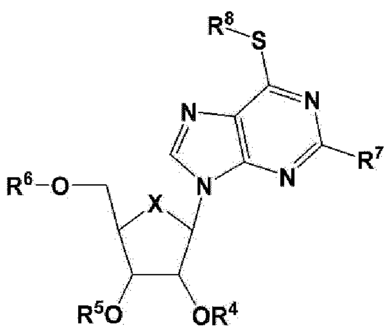

[2]

(In the formula, $R^4$ and $R^5$ are the same or different and represent a hydrogen atom or a hydroxyl protecting group; $R^6$ represents a hydrogen atom, a monophosphate group which may be protected, a diphosphate group which may be protected, or a triphosphoric acid group which may be protected; $R^7$ represents a hydrogen atom or an amino group which may be protected; and $R^8$ represents a hydrogen atom, a C1-6 alkyl group or a group represented by a formula [3]

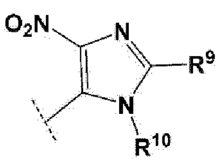

[3]

(In the formula, $R^9$ represents a hydrogen atom, a C1-6 alkyl group or a carboxy group; and $R^{10}$ represents a hydrogen atom, a C1-6 alkyl group, a benzyl group or a p-nitrobenzyl group.);

and X represents an oxygen atom, a sulfur atom, a carbon atom, or an imino group which may be protected.).

[2] The therapeutic agent according to [1], wherein $R^1$ is a hydrogen atom, $R^2$ is a fluorine atom or a hydrogen atom, and $R^3$ is a hydrogen atom.

[3] The therapeutic agent according to [1] or [2], wherein $R^1$ is a hydrogen atom, $R^2$ is a fluorine atom, and $R^3$ is a hydrogen atom.

[4] The therapeutic agent according to any one of [1] to [3], wherein $R^4$, $R^5$, $R^6$, and $R^7$ are each a hydrogen atom, $R^8$ is a methyl group, and X is an oxygen atom.

The present invention further provides the following:

[A] A method for treating RNA virus infection comprising administering to the subject a pyrazine derivative or a salt thereof represented by a formula [1]

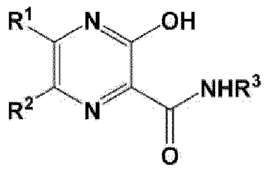 [1]

(In the formula, $R^1$ and $R^2$ are the same or different and represent a hydrogen atom or a halogen atom; and $R^3$ represents a hydrogen atom or an amino protecting group.) and a thiopurine derivative represented by a formula [2]

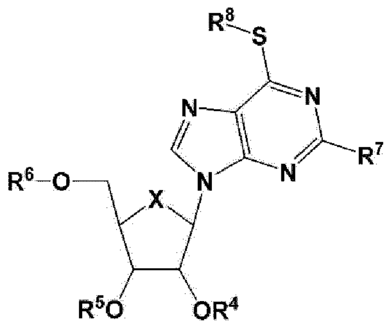 [2]

(In the formula, $R^4$ and $R^5$ are the same or different and represent a hydrogen atom or a hydroxyl protecting group; $R^6$ represents a hydrogen atom, a monophosphate group which may be protected, a diphosphate group which may be protected, or a triphosphoric acid group which may be protected; $R^7$ represents a hydrogen atom or an amino group which may be protected; and $R^8$ represents a hydrogen atom, a C1-6 alkyl group or a group represented by a formula [3].

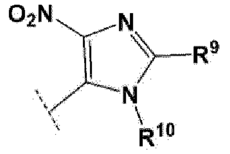 [3]

(In the formula, $R^9$ represents a hydrogen atom, a C1-6 alkyl group or a carboxy group; and $R^{10}$ represents a hydrogen atom, a C1-6 alkyl group, a benzyl group or a p-nitrobenzyl group.); and X represents an oxygen atom, a sulfur atom, a carbon atom, or an imino group which may be protected.).

[B] Use of a pyrazine derivative or a salt thereof represented by a formula [1]

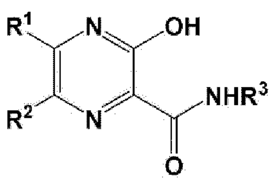 [1]

(In the formula, $R^1$ and $R^2$ are the same or different and represent a hydrogen atom or a halogen atom; and $R^3$ represents a hydrogen atom or an amino protecting group.) and a thiopurine derivative represented by the formula [2]

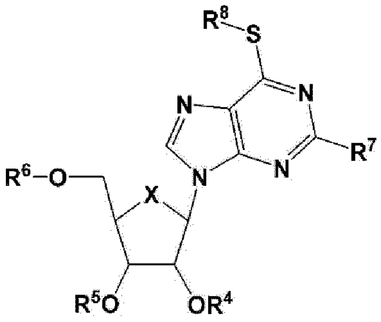

[2]

(In the formula, $R^4$ and $R^5$ are the same or different and represent a hydrogen atom or a hydroxyl protecting group; $R^6$ represents a hydrogen atom, a monophosphate group which may be protected, a diphosphate group which may be protected, or a triphosphoric acid group which may be protected; $R^7$ represents a hydrogen atom or an amino group which may be protected; and $R^8$ represents a hydrogen atom, a C1-6 alkyl group or a group represented by a formula [3]

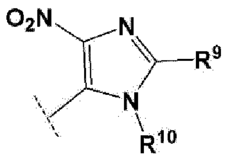

[3]

(In the formula, $R^9$ represents a hydrogen atom, a C1-6 alkyl group or a carboxy group; and $R^{10}$ represents a hydrogen atom, a C1-6 alkyl group, a benzyl group or a p-nitrobenzyl group.); and X represents an oxygen atom, a sulfur atom, a carbon atom, or an imino group which may be protected.) for production of a therapeutic agent for RNA virus infection.

[C] A combination of a pyrazine derivative or a salt thereof represented by a formula [1]

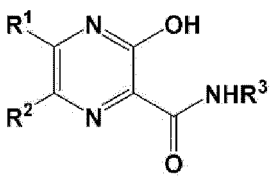

[1]

(In the formula, $R^1$ and $R^2$ are the same or different and represent a hydrogen atom or a halogen atom; and R3 represents a hydrogen atom or an amino protecting group.) and a thiopurine derivative represented by the formula [2]

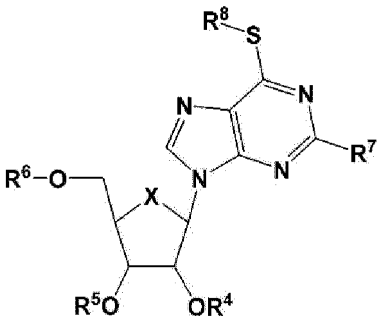

[2]

(In the formula, $R^4$ and $R^5$ are the same or different and represent a hydrogen atom or a hydroxyl protecting group; $R^6$ represents a hydrogen atom, a monophosphate group which may be protected, a diphosphate group which may be protected, or a triphosphoric acid group which may be protected; $R^7$ represents a hydrogen atom or an amino group which may be protected; and $R^8$ represents a hydrogen atom, a C1-6 alkyl group or a group represented by a formula [3]

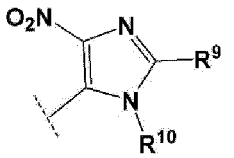

[3]

(In the formula, $R^9$ represents a hydrogen atom, a C1-6 alkyl group or a carboxy group; and $R^{10}$ represents a hydrogen atom, a C1-6 alkyl group, a benzyl group or a p-nitrobenzyl group.); and X represents an oxygen atom, a sulfur atom, a carbon atom, or an imino group which may be protected.) for use in the treatment of RNA virus infection.

## EFFECT OF THE INVENTION

A therapeutic agent for an RNA virus infection in which a pyrazine derivative or a salt thereof and a thiopurine derivative are combined is useful for treating or preventing RNA virus infection.

## FORM FOR CARRYING OUT THE INVENTION

The present invention will be described in detail below.

The halogen atom means fluorine atom, chlorine atom, bromine atom and iodine atom. The C1-6 alkyl group means a linear or branched C1-6 alkyl group such as methyl, ethyl, propyl, isopropyl, butyl, sec-butyl, isobutyl, tert-butyl, pentyl, isopentyl, 2-methylbutyl, 2-pentyl, 3-pentyl and hexyl group.

The amino protecting group includes all groups that can be used as a normal amino protecting group described in W. Greene et al., Protective Groups in Organic Synthesis, Fifth Version, 895-1193, 2014, John Wiley & Sons, Inc. etc.

Specifically, for example, an acyl group, an alkyloxycarbonyl group, an arylalkyloxycarbonyl group, an aryloxycarbonyl group, an arylalkyl group, an alkoxyalkyl group, an arylalkyloxyalkyl group, an arylthio group, an alkylsulfonyl group, an arylsulfonyl group, a dialkylaminoalkylidene group, an arylalkylidene group, a nitrogen-containing heterocyclic alkylidene group, a cycloalkylidene group, a diaryl phosphoryl group, a diaryl alkylphosphoryl group, an oxygen-containing heterocyclic alkyl group, and a substituted silyl group can be cited.

The imino-protecting group includes all groups that can be used as a protective group of an ordinary imino group described in W. Greene et al., Protective Groups in Organic Synthesis, Fifth Version, 895-1115, 2014, John Wiley & Sons, Inc. etc. Specifically, an ar C1-6 alkyl group, a C1-6 alkoxy C1-6 alkyl group, an acyl group, a C1-6 alkoxycarbonyl group, an ar C1-6 alkoxycarbonyl group, an aryloxycarbonyl group, a C1-6 alkylsulfonyl group, an arylsulfonyl group or a silyl group can be cited.

The hydroxyl protecting group includes all groups that can be used as protecting group of normal hydroxyl group described in w. Greene et al., Protective Groups in Organic Synthesis, fifth version, 17-471, 2014, John Wiley & Sons, Inc. etc.

Specifically, for example, a C1-6 alkyl group, an ar C1-6 alkyl group, a C1-6 alkoxy C1-6 alkyl group, an acyl group, a C1-6 alkoxycarbonyl group, a C1-6 alkoxycarbonyl group, a C1-6 alkylsulfonyl group, an arylsulfonyl group, a silyl group, a tetrahydrofuranyl group or a tetrahydropyranyl group can be cited.

The C1-6 alkyl group means a linear or branched C1-6 alkyl group such as methyl, ethyl, propyl, isopropyl, butyl, sec-butyl, isobutyl, tert-butyl, pentyl, isopentyl and hexyl groups.

The Ar C 1-6 alkyl group means an ar C1-6 alkyl group such as benzyl, diphenylmethyl, trityl, phenethyl and naphthylmethyl group.

The C1-6 alkoxy C1-6 alkyl group means a C1-6 alkyloxy C1-6 alkyl group such as methoxymethyl and 1-ethoxyethyl group

The acyl group means formyl group, c2-6 alkanoyl group, alloyl group or heterocyclic carbonyl group.

The C1-6 alkoxycarbonyl group means a linear or branched C1-6 alkyloxycarbonyl group such as methoxycarbonyl, ethoxycarbonyl, propoxycarbonyl, isopropoxycarbonyl, tert-butoxycarbonyl and 1,1-dimethylpropoxycarbonyl group.

The Ar C1-6 alkoxycarbonyl group means an ar C1-6 alkyloxycarbonyl group such as benzyloxycarbonyl and phenethyloxycarbonyl group.

The C1-6 alkylsulfonyl group means a C1-6 alkylsulfonyl group such as methylsulfonyl, ethylsulfonyl, and propyl sulfonyl group.

The arylsulfonyl group means benzenesulfonyl, p-toluenesulfonyl or naphthalene sulfonyl group. The silyl group means trimethylsilyl, triethylsilyl or tributyl silyl group.

Examples of a salt of the compound represented by formula [1] include commonly known salts in a hydroxyl group. For example, salts of alkali metals such as sodium and potassium; salts of alkaline earth metals such as calcium and magnesium; ammonium salts; and salts with nitrogen-containing organic nucleotides such as trimethylamine, triethylamine, tributylamine, N-methylpiperidine, N-methylmorpholine, diethyl amine, dicyclohexylamine, prokine, dibenzylamine, N-benzyl-beta-phenethyl amine, 1-ephemin and N,N'-dibenzylethylenediamine. As the preferable salt, a pharmacologically acceptable salt is cited, and a salt with sodium is more preferable.

In the compound of formula [1], $R^1$ is preferably a hydrogen atom; $R^2$ is a fluorine atom; and $R^3$ is a hydrogen atom. The compound is T -705, which is preferable compound.

Alternatively, in the compound of formula [1], $R^1$ is preferably a hydrogen atom; $R^2$ is a hydrogen atom; and $R^3$ is a hydrogen atom. The compound is T-1105, which is preferable compound.

The compound of formula [1] is manufactured by combining a known method, for example, the compound can be manufactured by the manufacturing method described in the pamphlet of International Publication No. 00/10569.

It is known that a pyrazine derivative represented by formula [1] or a salt thereof, for example, T-705 receives ribosyl phosphorylation in a cell, and a pyrazine derivative ribose triphosphate produced as a result shows an antiviral action (Antimicrob Agents Chemother. 2005; 49 (3): 981-6). Wherein the pyrazine derivative ribose triphosphate is represented by formula [4]

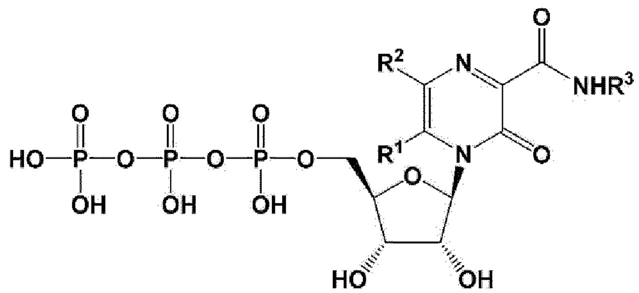

[4]

(In the formula, $R^1$ and $R^2$ are the same or different and represent a hydrogen atom or a halogen atom; and $R^3$ represents a hydrogen atom or an amino protecting group.).

In the compound of formula [2], $R^4$, $R^5$, $R^6$ and $R^7$ are preferably a hydrogen atom; $R^8$ is a methyl group; and X is an oxygen atom. The compound is 6-methylmercaptopurine riboside, which is preferable compound.

The combination of the compound of formula [1] and the compound of formula [2] is preferably a combination of T -705 and 6-methylmercaptopurine riboside.

The thiopurine derivative represented by formula [2] can enhance antiviral activity against an RNA virus of a pyrazine derivative represented by formula [1] or a salt thereof when used in combination with a pyrazine derivative represented by formula [1] or a salt thereof.

The thiopurine derivative represented by the formula [2] is used in combination with a pyrazine derivative represented by formula [1] or a salt thereof. When used in combination with a pyrazine derivative represented by formula [1] or a salt thereof, the amount of pyrazine derivative ribose triphosphate in a cell is increased as a result. Compared with a case where there is no combination, the concentration of the pyrazine derivative ribose triphosphate is preferably 1.5 times or more, more preferably 1.9 times or more. At least a part of the enhancing action of the antiviral activity increases the amount of the pyrazine derivative ribose triphosphate in the cell.

The thiopurine derivative may be produced by combining a known method itself or a commercially available one. For example, 6-methylmercaptopurine riboside can be purchased from Fuji Photo Film Wako Pure Chemical Co. Ltd.

In the present invention, a pyrazine derivative and a thiopurine derivative are used in combination. The combination includes a form of a pyrazine derivative and a pyrazine derivative at the same time, separately or in a specific order, and a form as a mixture (compounding agent).

That is, the period of administration of the pyrazine derivative or its salt and the thiopurine derivative is not only the same but also the form of administering the pyrazine derivative or its salt and the thiopurine derivative in one administration schedule is also included in the "combination". The administration route of the pyrazine derivative or its salt and the thiopurine derivative may be the same or different.

The amount ratio of the pyrazine derivative or the salt thereof and the thiopurine derivative may be an amount ratio in which the antiviral activity of the pyrazine derivative or the salt thereof is enhanced. Preferably, the pyrazine

derivative or its salt: thiopurine derivative (molar ratio) is 1: 500 to 500: 1, more preferably 1: 200 to 200: 1, more preferably 1: 50 to 50: 1, more preferably 1: 10 to 10: 1.

When a pyrazine derivative or a salt thereof and a thiopurine derivative of the present invention are used, a preparation auxiliary agent such as an excipient, a carrier, and a diluent used for formulation may be appropriately mixed, and these can be used in the form of a tablet, a capsule agent, a dispersant, a syrup agent, a granule, a round agent, a suspension, an emulsion, a powder preparation, a suppository, an eye drop, a point ear agent, a patch, an ointment or an injection agent according to a conventional method.

In the case of using as a mixture, a pyrazine derivative or a salt thereof and a thiopurine derivative are mixed in the preparation process and made uniform, and then a proper preparation may be used.

The administration route of the therapeutic agent for RNA virus infection of the present invention is not particularly limited, and can be administered intravenously, orally, intramuscular, subcutaneous, inhalation, spray, or other administration routes. The pyrazine derivative or its salt may be administered simultaneously with the thiopurine derivative or in a specific order.

Administration method, dosage, and number of times of administration can be appropriately selected according to age, weight and symptom of patient. usually, a pyrazine derivative as an active ingredient or a salt thereof is administered in an amount of 0.1-1,000 mg/kg, preferably 0.1-100 mg/kg per day, by administering to an adult by administration (eg, injection, intravenous drip and administration to a rectum site).

The therapeutic agent for RNA virus infection of the present invention is useful for treating or preventing RNA virus infection.

The RNA viral infectious disease is an infectious disease caused by an RNA virus such as an influenza virus, a perilla influenza virus, a bunya virus (Crimea Congo fever virus, Lift Valley fever virus, Lacrosse encephalitis virus, Dobrava virus, maporal virus, Prospecthill virus, Andean virus, Sandfly fever virus, Heartland virus, Puntatrovirus, Severe febrile thrombocytopenia syndrome virus, etc.), Arenavirus (funine virus, pitindevirus, tacaribe virus, guanaritovirus, macupovirus, lymphocytic choriomyelitis virus, Lassa fever virus, etc.), phyllovirus (Ebola virus, Marburg virus, etc.), mad dog disease virus, human metapneumovirus, RS virus, nipavirus, Hendra virus, measles virus, hepatitis A virus, hepatitis C virus, hepatitis E Virus, Chikunnya virus, Western horse encephalitis virus, Venezuelan encephalitis virus, Eastern horse encephalitis virus, Norovirus, Poliovirus, Echo virus, Coxsackie virus, Enterovirus, Rhinovirus, Rotavirus, Newcastle disease virus, Mumps virus, Bullous stomatitis virus, Japan Encephalitis virus, tick-borne flavivirus, yellow fever virus, dengue fever virus, western Nile virus, deca fever virus or coronavirus (SARS coronavirus, SARS coronavirus-2, MERS coronavirus, new coronavirus (SARS-CoV-2 and its mutant virus). The present invention can be used as a therapeutic agent for influenza virus, mad dog disease virus, Lassa fever virus, bunya virus (Crimea-Congo fever virus, Lift Valley fever virus, severe febrile thrombocytopenia syndrome virus, etc.) and phyllovirus (Ebola virus, Marburg virus, etc.), and particularly preferably as a therapeutic agent for influenza virus infection.

The therapeutic agent for RNA virus infection of the present invention can be used in combination with other RNA virus infection therapeutic agent or an anti-RNA virus-inhibiting agent for the purpose of enhancing the action or the like. The other therapeutic agent for RNA virus infection includes, for example, an influenza therapeutic agent, a therapeutic agent for hepatitis C, a therapeutic agent for filovirus infection, and the like. Examples of the influenza therapeutic agent include amantadine, rimantadine, oseltamivir, zanamivir, peramivir, laninamivir, baloxavir and the like. Examples of the therapeutic agent for hepatitis C include ribavirin, pegged interferon, telaprevir, boceprevir, galidesivir and the like. Examples of therapeutic agents for phyllovirus infections include ribavirin, paribizumab, motabizumab, RSV-IGIV, MEDI-557, A-60444, MDT-637, BMS-433771, amiodaron, dronedaron, verapamil, and Ebola convalescent plasma, TKM-100201, BCX4430, FGI-106, TKM-Ebola, ZMapp, rNAPc2, OS-2966, MVA-BNfilo, Brinsidefobil, Ad26-ZEBOV and the like. Examples of the drug exhibiting anti-RNA virus inhibitory action include mycophenolic acid, daptomycin, nicorosamide, azithromycin, novobiocin, chloroquine, memantine, prochlorperazine, chlorcyclidine, manidipine, remdesivir, Imatinib, chlorpromazine, nitazoxanide and the like (Journal of Young Pharmacists. 2019; 11 (2): 117-121.).

EXAMPLES

Next, the present invention will be described with reference to Examples, but the present invention is not limited thereto.

Test Example 1

Combination effect of pyrazine derivative and thiopurine derivative was tested by using virus cell infection model

T-705 was selected as pyrazine derivative. 6-methylmercaptopurine riboside was selected as a thiopurine derivative. Influenza viruse was selected as RNA viruses.

(1) Culturing Vero cells African green monkey kidney Vero cell culture which were subcultured at 37 °C. under 5%

carbon dioxide conditions in a culture medium containing 10% bovine fetal serum-added eagle MEM / canamycin 60 μg / mL (eagle MEM / canamycin) were exfoliated by the trypsin method of ethylenediamine tetraacetate, and a suspension prepared to contain $2 \times 10^4$ cells in 100 μL in the same medium was seeded on a 96-well plate. Under 5% carbon dioxide conditions, the cells were cultured overnight at 37 ° C to obtain monolayered Vero cells.

(2) Influenza virus infection and chemical addition

As a test medium, a medium prepared by adding L-1-tosylamide-2-phenylethylchloromethylketone (TPCK) -treated trypsin to an eagle MEM / kanamycin medium at 1 μg / mL was used. The culture supernatant of Vero cells obtained in (1) was removed, and the following (A) to (C) were added to each well. After the addition of the drug, the cells were cultured at 35 ° C for 2 days under 5% carbon dioxide conditions.

(A) 100 μL of Eagle MEM / Kanamycin medium

(B) 50 μL of influenza virus (PR / 8/34 (H1N1)) solution adjusted to $4.0 \times 10^3$ PFU / mL with Eagle MEM / kanamycin medium containing 4 times the concentration of TPCK-treated trypsin as the test medium.

(C) 50 μL of 1% DMSO-containing Eagle MEM / kanamycin medium containing a combination of each set concentration of T-705 and 6-methylmercaptopurine riboside at 4 times the set concentration.

T-705 set concentration (μM):
0,0.1,0.3,1,3,10,30,100,300,1000

6-Methylmercaptopurine riboside set concentration (μM):
0,0.1,0.3,1,3,10

(3) Determination of a cell modification effect (CPE)

The CPE observed with the growth of influenza virus was determined by the following method.

After the culture was completed, 50 μL of 100% formalin solution was added to each well to inactivate the virus and fix the cells. After allowing to stand at room temperature for 2 hours or more, the aqueous solution was removed, the mixture was lightly washed with water, 50 μL / well of 0.02% methylene blue solution was added, and the mixture was allowed to stand at room temperature for 1 hour. The 0.02% methylene blue solution was removed, lightly washed with water, and then air-dried. Then, the absorbance (660 nm) was measured with a microplate reader (manufactured by Tecan, infinit M200). For non-infection control, 50 μL of Eagle MEM / canamycin medium containing TPCK-treated trypsin at a concentration 4 times that of the test medium was added instead of the influenza virus solution, and the same operation as in the test group was performed to measure the absorbance.

The test was carried out with 1 / plate of cases and 2 plates (8 cases of infection control and non-infection control). The value obtained by subtracting the absorbance of the infection control from the absorbance of the non-infection control was taken as the complete suppression value of virus growth, and the CPE inhibition rate of each test was calculated from the formula shown below.

CPE inhibition rate = 100 x [(absorbance during single agent and combination action)-(absorbance of infection control)] / [(absorbance of non-infection control)-(absorbance of infection control)]

The FORECAST function (first-order regression method) of Microsoft Office Excel 2010 was used to calculate the 50% CPE inhibition concentration.

Table 1 shows the 50% CPE inhibitory concentration change of T-705 when 6-methylmercaptopurine riboside was added. The 50% CPE inhibitory concentration of T-705 was lower in combination with 6-methylmercaptopurine riboside as compared to T-705 alone. In this test system, 6-methylmercaptopurine riboside (10 μM) alone did not show a CPE inhibitory effect.

[table 1]

| The 50% CPE inhibitory concentration change of T-705 by adding 6-methylmercaptopurine riboside | | | | | | |
|---|---|---|---|---|---|---|
| Concentration of 6-methylmercaptopurine (μM) | 0 | 0.1 | 0.3 | 1 | 3 | 10 |
| The 50% CPE inhibitory concentration of T-705 (μM) | 183 | 89.4 | 48.9 | 30.6 | 21.9 | 20.2 |

It was confirmed in the cell infection model that the antiviral activity was enhanced by the combination of T-705 and 6-methylmercaptopurine riboside as compared with the case of T-705 alone.

TEST EXAMPLE 2

As mentioned above, pyrazine derivatives or salts thereof undergo intracellular ribosyl phosphorylation. For example, T-705 is known to exhibit antiviral activity by inhibiting the viral RdRp protein by T-705-4-ribofuranosyll-5-triphosphate (T-705RTP) produced by ribosyl phosphorylation. In order to confirm whether the activity-enhancing effect of T-705 by 6-methylmercaptopurine riboside seen in Test Example 1 is due to a change in the amount of T-705RPP, the amount of T-705RTP below was measured under 6-Methylmercaptopurine riboside treatment in Vero cells.

(1) Measurement of T-705RTP amount in Vero cells under treatment with 6-methylmercaptopurineriboside

(1-1) Extraction of intracellular T-705RPP

Vero cells which were seeded in each well of a 6-well plate at 2 × $10^5$ cells were cultured in vehicle (0.0125% DMSO) or various concentrations of 6-methylmercaptopurineriboside, 100 μM T-705 or both agents under 5% carbon dioxide conditions at 37 ° C for 24 hours (Example 3).

After culturing, the cells were washed with PBS, 300 μL trypsin was added, and the cells were allowed to stand at 37 ° C for 5 minutes to peel off the cells. 700 μL of Eagle's MEM medium supplemented with 10% fetal bovine serum was added, suspended, and collected in a 1.5 mL tube. Of that, 10 μL was used for cell number measurement.

All remaining cell suspensions were centrifuged at 1,000 rpm, 4 ° C for 5 minutes (MX-207, TOMY) to remove the supernatant. Next, 1 mL PBS was added to the cell pellet, the cells were resuspended, and the cells were centrifuged again at 1,000 rpm at 4 ° C for 5 minutes, and the supernatant was removed. Then, 300 μL of 70% methanol was added and suspended well. Samples were then measured using a high performance liquid chromatograph (HPLC-FL) with a fluorescence detector.

(1-2) Measurement of cell number and HPLC-FL analysis

The cell number was measured and HPLC-FL analysis was performed, and the amount of T-705RTP in each sample was measured. 20 μL of 6-bromo-3-hydroxy-2-pyrazinecarboxamide solution and 180 μL of distilled water as internal standards were added to 50 μL of a well-stirred cell sample (prepared in (1-1) above) under ice cooling and the mixture was centrifuged (about 1600 rpm, room temperature, 5 minutes, MICRO SINKER) and the supernatant was analyzed by HPLC. A C18 column was used for HPLC analysis, and T-705RTP was retained in the column by adding an ion pair reagent (tetrabutylammonium bromide) to the weakly acidic mobile phase. The excitation wavelength for fluorescence detection was 370 nm, and the fluorescence wavelength was 445 nm. From the HPLC area ratio (T-705RTP / internal standard) of each cell sample, the T-705RTP concentration was determined by the reverse regression of the calibration curve. Table 2 shows changes in the amount of T-705RTP. It was found that 6-methylmercaptopurine riboside increased the intracellular T-705RTP amount after T-705 treatment.

[Table 2]

| The change of T-705RTP amount in Vero cells | | | |
|---|---|---|---|
| Concentration of 6-methylmercaptopurine (μM) | 0 | 1 | 10 |
| T-705RTP concentration (pmol/$10^6$ cells) | 88.3 | 148 | 167 |

The mechanism of action of T-705, that is, that it exhibits antiviral activity after being converted to T-705RTP in cells, is applicable to any virus as long as T-705 is effective. Since the intracellular T-705RTP amount was increased by the thiopurine derivative, it can be said that the combination of T-705 and the thiopurine derivative simultaneously enhances the antiviral activity against a plurality of RNA viruses for which T-705 is effective.

This mechanism applies not only to T-705 but also to a pyrazine derivative represented by the formula [1] or a salt thereof. For example, in addition to T-705, it is suggested that the compound (3-hydroxy-pyrazincarboxamide) of formula [1] in which $R^1$ is a hydrogen atom; $R^2$ is a hydrogen atom; and $R^3$ is a hydrogen atom shows antivirus activity (Antival Res. 2009; 82 (3): 95-102.) and is intracellularly transformed into a ribose triphosphate (Mol Pharmacol. 2013; 84 (4): 615-29.). That is, by combining a pyrazine derivative or a salt thereof with a thiopurine derivative, the antiviral activity against a plurality of RNA viruses to which the pyrazine derivative or the salt thereof is effective can be simultaneously enhanced.

## INDUSTRIAL APPLICABILITY

An RNA virus infection therapeutic agent comprising a combination of a pyrazine derivative or a salt thereof and a thiopurine derivative exhibits enhanced antiviral activity and is useful in the field of pharmaceutical industry.

## Claims

1. A therapeutic agent for an RNA virus infection comprising a combination of a pyrazine derivative or a salt thereof represented by a formula [1]

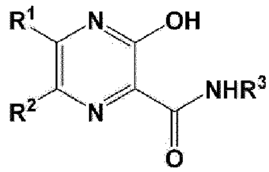

[1]

(In the formula, $R^1$ and $R^2$ are the same or different and represent a hydrogen atom or a halogen atom; and $R^3$ represents a hydrogen atom or an amino protecting group.) and a thiopurine derivative represented by a formula [2]

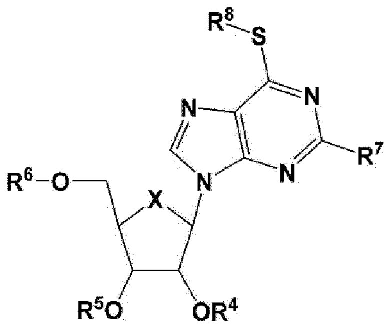

[2]

(In the formula, $R^4$ and $R^5$ are the same or different and represent a hydrogen atom or a hydroxyl protecting group; $R^6$ represents a hydrogen atom, a monophosphate group which may be protected, a diphosphate group which may be protected, or a triphosphoric acid group which may be protected; $R^7$ represents a hydrogen atom or an amino group which may be protected; and $R^8$ represents a hydrogen atom, a C1-6 alkyl group or a group represented by formula [3]

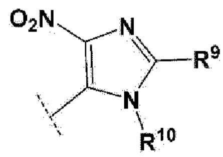

[3]

(In the formula, $R^9$ represents a hydrogen atom, a C1-6 alkyl group or a carboxy group; and $R^{10}$ represents a hydrogen atom, a C1-6 alkyl group, a benzyl group or a p-nitrobenzyl group.); and X represents an oxygen atom, a sulfur atom, a carbon atom, or an imino group which may be protected.).

2. The therapeutic agent according to claim 1, wherein $R^1$ is a hydrogen atom, $R^2$ is a fluorine atom or a hydrogen atom, and $R^3$ is a hydrogen atom.

3. The therapeutic agent according to claim 1 or 2, wherein $R^1$ is a hydrogen atom, $R^2$ is a fluorine atom, and $R^3$ is a hydrogen atom.

4. The therapeutic agent according to any one of claim 1 to 3, wherein $R^4$, $R^5$, $R^6$, and $R^7$ are each a hydrogen atom, $R^8$ is a methyl group, and X is an oxygen atom.

## INTERNATIONAL SEARCH REPORT

International application No. PCT/JP2021/019918

A. CLASSIFICATION OF SUBJECT MATTER
A61K 31/4965(2006.11)i; A61K 31/7076(2006.01)i; A61P 31/14(2006.01)i; A61P 43/00(2006.01)i; C07D 241/24(2006.01)i
FI: A61K31/4965; A61K31/7076; A61P31/14; A61P43/00 121; C07D241/24
According to International Patent Classification (IPC) or to both national classification and IPC

B. FIELDS SEARCHED
Minimum documentation searched (classification system followed by classification symbols)
A61K31/4965; A61K31/7076; A61P31/14; A61P43/00; C07D241/24

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII); CAplus/REGISTRY/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| Y | FURUTA, Y. et al., "T-705 (favipiravir) and related compounds: Novel broad-spectrum inhibitors of RNA viral infections", Antiviral Research, vol. 82, 2009, pp. 95-102 abstract, page 96, right column, line 1 to page 97, right column, line 2, page 99, right column, line 27 to page 100, right column, line 13, page 101, left column, lines 44-50, fig. 1, etc. | 1-4 |
| Y | HOOVER, S. et al., "Thiopurines inhibit bovine viral diarrhea virus production in a thiopurine methyl transferase-dependent manner", Journal of General Virology, vol. 89, 2008, pp. 1000-1009 abstract, page 89, right column, lines 10-28, fig. 1, 2, 3 | 1-4 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 July 2021 (07.07.2021) | 20 July 2021 (20.07.2021) |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No.
PCT/JP2021/019918

C (Continuation). DOCUMENTS CONSIDERED TO BE RELEVANT

| Category* | Citation of document, with indication, where appropriate, of the relevant passages | Relevant to claim No. |
|---|---|---|
| A | WO 2008/099874 A1 (TOYAMA CHEMICAL CO., LTD.) 21 August 2008 (2008-08-21) claims, test example 1, etc. | 1-4 |
| A | GOVORKOVA, E. A. et al., "Neuraminidase Inhibitor-Rimantadine Combinations Exert Additive and Synergistic Anti-Influenza Virus Effects in MDCK Cells", ANTIMICROBIAL AGENTS AND CHEMOTHERAPY, 2004, vol. 48, no. 12, pp. 4855-4863 title, abstract | 1-4 |
| A | SHIGETA, S., "Recent Progress in Anti-Influenza Chemotherapy", Drugs R&D, vol. 2, no. 3, 1999, pp. 153-164 abstract | 1-4 |
| A | MADREN, L. K. et al., "In vitro inhibitory effects of combinations of anti-influenza agents", Antiviral Chemistry & Chemotherapy, vol. 6, no. 2, 1995, pp. 109-113 abstract | 1-4 |
| Y<br>A | DESPINS, S. et al., "Deciphering the molecular basis for nucleotide selection by the West Nile virus RNA helicase", Nucleic Acids Research, vol. 38, no. 16, 2010, pp. 5493-5506 abstract, page 5493, left column, lines 22-26, table 1, fig. 3 | 1-3<br>4 |

**INTERNATIONAL SEARCH REPORT**
Information on patent family members

International application No.
PCT/JP2021/019918

| Patent Documents referred in the Report | Publication Date | Patent Family | Publication Date |
|---|---|---|---|
| WO 2008/099874 A1 | 21 Aug. 2008 | US 2010/0087447 A1<br>claims, test example 1<br>EP 2123276 A1<br>CN 101610772 A<br>KR 10-2009-0121289 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 08099874 A
- WO 2007202789 A
- WO 0010569 A


### Non-patent literature cited in the description


- *Proc Jpn Acad Ser B Phys Biol Sci,* vol. 93, 449-463
- **CDC HEALTH ADVISORY.** *CDC issues interim recommendations for the use of influenza antivirals in the setting of oseltamivir Resistance among circulating influenza A (H1N1) viruses,* 2008
- *PLoS One,* October 2011, vol. 6, e26697
- **W. GREENE et al.** Protective Groups in Organic Synthesis. John Wiley & Sons, Inc, 2014, 895-1193
- **W. GREENE et al.** Protective Groups in Organic Synthesis. John Wiley & Sons, Inc, 2014, 895-1115
- **GREENE et al.** Protective Groups in Organic Synthesis. John Wiley & Sons, Inc, 2014, 17-471
- *Antimicrob Agents Chemother.,* 2005, vol. 49 (3), 981-6
- *Journal of Young Pharmacists,* 2019, vol. 11 (2), 117-121
- *Antival Res.,* 2009, vol. 82 (3), 95-102
- *Mol Pharmacol.,* 2013, vol. 84 (4), 615-29